# EUROPEAN PATENT SPECIFICATION

(11) **EP 1 846 390 B1**
(45) Date of publication and mention of the grant of the patent: **17.03.2010**
(21) Application number: 06707926.9
(22) Date of filing: 31.01.2006
(51) Int. Cl.: C07D 401/04, A61K 31/473, A61P 11/06

(54) **NOVEL 6-PYRIDYLPHENANTHRIDINES**
NEUE 6-PYRIDYLPHENANTHRIDINE
NOUVELLES 6-PYRIDYLPHENANTHRIDINES

(30) Priority: 01.02.2005 EP 05100690
(43) Date of publication of application: 24.10.2007
(73) Proprietor: Nycomed GmbH, 78467 Konstanz (DE)
(72) Inventor: KAUTZ, Ulrich, 78476 Allensbach (DE); SCHMIDT, Beate, 78476 Allensbach (DE); FLOCKERZI, Dieter, 78476 Allensbach (DE); CHIESA, Maria Vittoria, Biberach an der Riss (DE); HATZELMANN, Armin, 78467 Konstanz (DE); ZITT, Christof, 78464 Konstanz (DE); BARSIG, Johannes, 78467 Konstanz (DE); WOHLSEN, Andrea, 2000 Neuchâtel (CH); MARX, Degenhard, 78345 Moos (DE); KLEY, Hans-peter, 78476 Allensbach (DE)
(74) Representative: Mechnich, Oliver M.J.
(86) International application number: PCT/EP2006/050557
(87) International publication number: WO 2006/082185

(56) References cited:
- WO-A-97/35854

## Description

### Field of application of the invention

The invention relates to novel 6-pyridylphenanthridines, which are used in the pharmaceutical industry for the production of pharmaceutical compositions.

### Technical background

The international application WO 97/35854 describes 6-pyridylphenanthridines as PDE4 inhibitors.

The international application WO 2005/085225 describes hydroxyl-6-heteroarylphenanthridines as PDE4 inhibitors.

### Description of the invention

It has now been found that the novel 6-pyridylphenanthridines, which are described in greater detail below and differ from the previously known 6-pyridylphenanthridines by unanticipated substitution patterns on the 6-pyridyl ring, have surprising and particularly advantageous properties.

The invention thus relates to compounds of the formula I, 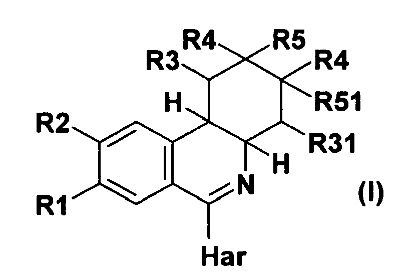 in which
- R1: is methoxy or ethoxy,
- R2: is methoxy, ethoxy or difluoromethoxy,
- R3, R31, R4, R5 and R51: are hydrogen,
- Har: is selected from 2,6-dimethoxypyridin-4-yl, 2,6-dimethoxypyridin-3-yl, 4,6-dimethoxy-pyridin-3-yl, 4,6-diethoxy-pyridin-3-yl, 5,6-dimethoxy-pyridin-3-yl, 5-ethoxy-6-methoxy-pyridin-3-yl and 1-methyl-1H-pyridin-2-one-5-yl,
and the salts, the N-oxides and the salts of the N-oxides of these compounds.

As it is known for the person skilled in the art, compounds comprising nitrogen atoms can be form N-oxides. Particularly, imine nitrogen, especially heterocyclic or heteroaromatic imine nitrogen, or pyridine-type nitrogen (=N-) atoms, can be N-oxidized to form the N-oxides comprising the group =N⁺(O⁻)-. Thus, the compounds according to the present invention comprising the imine nitrogen atom in position 5 of the phenylphenanthridine backbone and, optionally (depending on the meaning of the substituents), one or more further nitrogen atoms suitable to exist in the N-oxide state (=N⁺(O⁻)-) may be capable to form (depending on the number of nitrogen atoms suitable to form stabile N-oxides) mono-N-oxides, bis-N-oxides or multi-N-oxides, or mixtures thereof.

The term N-oxide(s) as used in this invention therefore encompasses all possible, and in particular all stabile, N-oxide forms, such as mono-N-oxides, bis-N-oxides or multi-N-oxides, or mixtures thereof in any mixing ratio.

Possible salts for compounds of the formula I -depending on substitution- are all acid addition salts or all salts with bases. Particular mention may be made of the pharmacologically tolerable salts of the inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 3-hydroxy-2-naphthoic acid, it being possible to employ the acids in salt preparation - depending on whether a mono- or polybasic acid is concerned and depending on which salt is desired - in an equimolar quantitative ratio or one differing therefrom.

On the other hand, salts with bases are also suitable. Examples of salts with bases which may be mentioned are alkali metal (lithium, sodium, potassium) or calcium, aluminum, magnesium, titanium, ammonium, meglumine or guanidinium salts, where here too the bases are employed in salt preparation in an equimolar quantitative ratio or one differing therefrom.

Pharmacologically intolerable salts which can initially be obtained, for example, as process products in the preparation of the compounds according to the invention on an industrial scale are converted into pharmacologically tolerable salts by processes known to the person skilled in the art.

It is known to the person skilled in the art that the compounds of formula I according to the invention and their salts, when they are isolated, for example, in crystalline form, can contain various amounts of solvents. The invention therefore also comprises all solvates and in particular all hydrates of the compounds of the formula I, and also all solvates and in particular all hydrates of the salts of the compounds of the formula I.

Furthermore, the invention includes all conceivable tautomeric forms of the compounds of the present invention in pure form as well as any mixtures thereof. In this connection, the person skilled in the art knows that enolizable keto groups can exist, depending on the individual chemical surrounding, in their tautomeric enol forms, and vice versa. As it is art-known hereby, keto and enol functions can mutually exchange in equilibrium. The invention includes in this context both the stable keto and the stable enol isomers of the compounds according to this invention in pure form, as well as the mixtures thereof, in any mixing ratio.

Compounds of formula I according to this invention in particular worthy to be mentioned are (4aR, 10bR)-6-(2,6-Dimethoxy-pyridin-3-yl)-8,9-dimethoxy-1,2,3,4,4a,10b-hexahydro-phenanthridine, (4aR,10bR)-6-(2,6-Dimethoxy-pyridin-3-yl)-9-ethoxy-8-methoxy-1,2,3,4,4a,10b-hexahydrophenanthridine, and the salts, the N-oxides and the salts of the N-oxides of these compounds.

The compounds of the formula I are chiral compounds having chiral centers at least in positions 4a and 10b.Numbering:

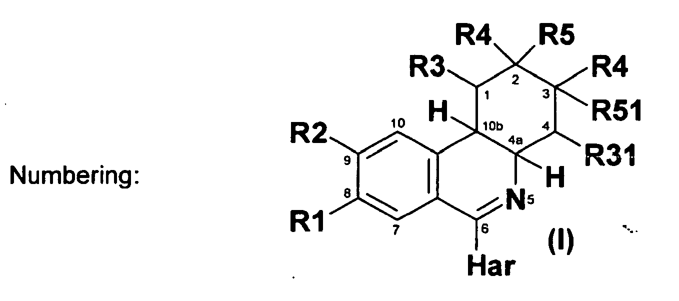

The invention therefore comprises all conceivable stereoisomers in pure form as well as in any mixing ratio, and the salts thereof.

Preferred compounds of the formula I are those in which the hydrogen atoms in positions 4a and 10b are in the cis position relative to one another. The pure cis diastereomers, the pure cis enantiomers and their mixtures in any mixing ratio and including the racemates are more preferred in this context.

Particularly preferred in this connection are those compounds of the formula I which have, with respect to the positions 4a and 10b, the same configuration as shown in the formula I*:

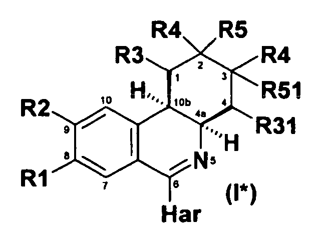

If, for example in compounds of the formula I* R3, R31, R4, R5 and R51 have the meaning hydrogen, then the configuration - according the rules of Cahn, Ingold and Prelog - is R in the position 4a and R in the position 10b.

The enantiomers can be separated in a manner known per se (for example by preparation and separation of appropriate diastereoisomeric compounds). For example, an enantiomer separation can be carried out at the stage of the starting compounds of the formula IV in which R1, R2, R3, R31, R4, R5 and R51 have the meanings indicated above.

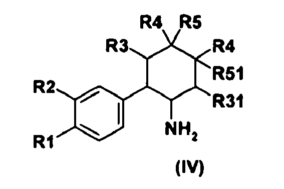

Separation of the enantiomers can be carried out, for example, by means of salt formation of the racemic compounds of the formula IV with optically active acids, preferably carboxylic acids, subsequent resolution of the salts and release of the desired compound from the salt. Examples of optically active carboxylic acids which may be mentioned in this connection are the enantiomeric forms of mandelic acid, tartaric acid, O,O'-dibenzoyltartaric acid, camphoric acid, quinic acid, glutamic acid, malic acid, camphorsulfonic acid, 3-bromocamphorsulfonic acid, α-methoxyphenylacetic acid, α-methoxy-α-trifluoromethylphenylacetic acid and 2-phenylpropionic acid. Alternatively, enantiomerically pure starting compounds of the formula IV can be prepared via asymmetric syntheses. Enantiomerically pure starting compounds as well as enantiomerically pure compounds of the formula I can be also obtained by chromatographic separation on chiral separating columns; by derivatization with chiral auxiliary reagents, subsequent diastereomer separation and removal of the chiral auxiliary group; or by (fractional) crystallization from a suitable solvent.

Compounds of the formula I, in which R1, R2, R3, R31, R4, R5, R51 and Har have the meanings indicated above, can be prepared according to those procedures given by way of example in the following examples. For greater detail, a suitable synthesis route for compounds of the formula I is outlined in reaction scheme 1 below. In the first step of said reaction scheme 1 compounds of the formula IV, in which R1, R2, R3, R31, R4, R5 and R51 have the meanings given above, are reacted with compounds of the formula III, in which Har has the meanings given above and X represents a suitable leaving group, preferably a chlorine atom, to give compounds of the formula II, in which R1, R2, R3, R31, R4, R5, R51 and Har have the abovementioned meanings.

Alternatively, compounds of the formula II, in which R1, R2, R3, R31, R4, R5, R51 and Har have the meanings given above, can also be prepared, for example, from compounds of the formula IV, in which R1, R2, R3, R31, R4, R5 and R51 have the abovementioned meanings, and compounds of the formula III, in which Har has the abovementioned meanings and X is hydroxyl, by reaction with amide bond linking reagents known to the person skilled in the art. Exemplary amide bond linking reagents known to the person skilled in the art which may be mentioned are, for example, the carbodiimides (e.g. dicyclohexylcarbodiimide or, preferably, 1-ethyl-3-(3-dimethylaminopropyl)carbodiimide hydrochloride), azodicarboxylic acid derivatives (e.g. diethyl azodicarboxylate), uronium salts [e.g. O-(benzotriazol-1-yl)-N,N,N',N'-tetramethyluronium tetrafluoroborate or O-(benzotriazol-1yl)-N,N,N',N'-tetramthyl-uronium-hexafluorophosphate] and N,N'-carbonyldiimidazole. In the scope of this invention preferred amide bond linking reagents are uronium salts and, particularly, carbodiimides, preferably, 1-ethyl-3-(3-dimethylaminopropyl)carbodiimide hydrochloride.

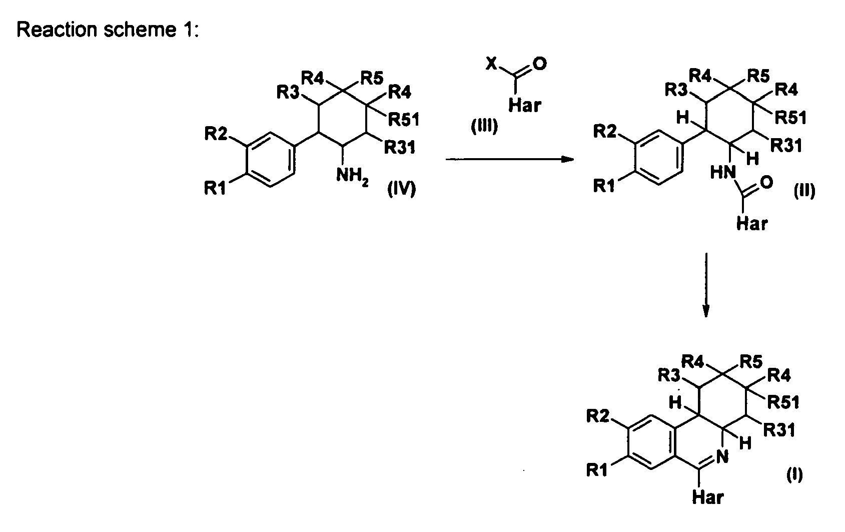

Compounds of the formula III are either known or can be prepared in according to known procedures.

As shown in the next step within reaction scheme 1, compounds of the formula I, in which R1, R2, R3, R31, R4, R5, R51 and Har have the meanings indicated above, can be obtained by cyclocondensation of corresponding compounds of the formula II. Said cyclocondensation reaction is carried out in a manner habitual per se to the person skilled in the art or as described by way of example in the following examples, according to Bischler-Napieralski (e.g. as described in J. Chem. Soc., 1956, 4280-4282) in the presence of a suitable condensing agent, such as, for example, polyphosphoric acid, phosphorus pentachloride, phosphorus pentoxide or phosphorus oxychloride, in a suitable inert solvent, e.g. in a chlorinated hydrocarbon such as chloroform, or in a cyclic hydrocarbon such as toluene or xylene, or another inert solvent such as acetonitrile, or without further solvent using an excess of condensing agent, at reduced temperature, or at room temperature, or at elevated temperature or at the boiling temperature of the solvent or condensing agent used.
If necessary, said cyclocondensation reaction can be carried out in the presence of one or more suitable Lewis Acids such as, for example, suitable metal halogenides (e.g. chlorides) or sulphonates (e.g. triflates), including rare earth metal salts, such as e.g. anhydrous aluminum trichloride, aluminum tribromide, zinc chloride, boron trifluoride ethereate, titanium tetrachloride or, in particular, tin tetrachloride, and the like. Parallel to the cyclization in the presence of a chlorine-containing condensing agent (such as e.g. phosphorus pentachloride), a nucleophilic or electrophilic substitution of the Har moiety giving the corresponding chlorine substituted Har moiety can take place, especially in the case of electron rich Har groups, such as e.g. the dimethoxypyridinyl radical, like the 2,6-dimethoxypyridin-4-yl or the 2,6-dimethoxy-pyridin-3-yl radical, an electrophilic substitution can take place.

The preparation of pure enantiomeres of starting compounds of the formula IV can be carried out as described, for example, in the international application WO00/42020 or in a manner according to the following examples.

In addition, the compounds of the formula I can be converted, optionally, into their N-oxides, for example with the aid of hydrogen peroxide in methanol or with the aid of m-chloroperoxybenzoic acid in dichloromethane. The person skilled in the art is familiar on the basis of his/her expert knowledge with the reaction conditions which are specifically necessary for carrying out the N-oxidation.

It is moreover known to the person skilled in the art that if there are a number of reactive centers on a starting or intermediate compound it may be necessary to block one or more reactive centers temporarily by protective groups in order to allow a reaction to proceed specifically at the desired reaction center. A detailed description for the use of a large number of proven protective groups is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

The substances according to the invention are isolated and purified in a manner known per se, for example by distilling off the solvent under reduced pressure and recrystallizing the residue obtained from a suitable solvent or subjecting it to one of the customary purification methods, such as, for example, column chromatography on a suitable support material.

Salts are obtained by dissolving the free compound in a suitable solvent (e.g. a ketone, such as acetone, methyl ethyl ketone or methyl isobutyl ketone, an ether, such as diethyl ether, tetrahydrofuran or dioxane, a chlorinated hydrocarbon, such as methylene chloride or chloroform, or a low-molecular-weight aliphatic alcohol, such as methanol, ethanol or isopropanol) which contains the desired acid or base, or to which the desired acid or base is then added. The salts are obtained by filtering, reprecipitating, precipitating with a nonsolvent for the addition salt or by evaporating the solvent. Salts obtained can be converted into the free compounds, which can in turn be converted into salts, by alkalization or by acidification. In this manner, pharmacologically unacceptable salts can be converted into pharmacologically acceptable salts.

Optionally, compounds according to this invention can be converted into their salts, or, optionally, salts of the compounds according to this invention can be converted into the free compounds.

Suitably, the conversions mentioned in this invention can be carried out analogously or similarly to methods which are familiar per se to the person skilled in the art.

The person skilled in the art knows on the basis of his/her knowledge and on the basis of those synthesis routes, which are shown and described within the description of this invention, how to find other possible synthesis routes for compounds of the formula I. All these other possible synthesis routes are also part of this invention.

Having described the invention in detail, the scope of the present invention is not limited only to those described characteristics or embodiments. As will be apparent to persons skilled in the art, modifications, analogies, variations, derivations, homologisations and adaptations to the described invention can be made on the base of art-known knowledge and/or, particularly, on the base of the disclosure (e.g. the explicite, implicite or inherent disclosure) of the present invention without departing from the spirit and scope of this invention as defined by the scope of the appended claims.

The following examples serve to illustrate the invention further without restricting it. Likewise, further compounds of the formula I, whose preparation is not explicitly described, can be prepared in an analogous or similar manner or in a manner familiar per se to the person skilled in the art using customary process techniques.

Any or all of the compounds of formula I which are mentioned in the following examples as well as their salts, N-oxides and salts of the N-oxides are a preferred subject of the present invention.

In the examples, m.p. stands for melting point, h for hour(s), min for minutes, R*_{f}* for rentention factor in thin layer chromatography, s.p. for sintering point, EF for empirical formula, MW for molecular weight, MS for mass spectrum, M for molecular ion, fnd. for found, calc. for calculated, other abbreviations have their meanings customary per se to the skilled person.

### Examples

### Final products:

**1. (4aR,10bR)-6-(2,6-Dimethoxy-pyridin-3-yl)-8,9-dimethoxy-1,2,3,4,4a,10b-hexahydrophenanthridine**
The crude N-[(1R,2R)-2-(3,4-dimethoxy-phenyl)-cyclohexyl]-2,6-dimethoxy-nicotinamide (compound A1) is dissolved in 15 ml of dichloromethane and 8.48 g of phosphorus pentachloride are added. After stirring for 16 h and work-up (sodium hydroxide / water) the residue is purified by means of chromatography to yield 0.747 g of the title compound.

| | | | | |
|---|---|---|---|---|
| MS: | calc.: | C₂₂ H₂₆ N₂ O₄ | (382.46) | fnd.: [M+H] 383.3 |

**2. (4aR,10bR)-6-(2,6-Dimethoxy-pyridin-3-yl)-9-ethoxy-8-methoxy-1,2,3,4,4a,10b-hexahydrophenanthridine**
Starting from compound A2 the title compound can be obtained analogously as described for Example 1.

| | | | | |
|---|---|---|---|---|
| MS: | calc.: | C₂₃ H₂₈ N₂ O₄ | (396.49) | fnd.: [M+H] 397.3 |

Using similar procedures to those to attain to Example 1, but with suitable choice of starting materials (which can be prepared according to procedures which are customary to the skilled person or which are described herein, or analogously or similarly thereto), the following compounds may be prepared:
(4aR,10bR)-6-(4,6-Dimethoxy-pyridin-3-yl)-9-ethoxy-8-methoxy-1,2,3,4,4a,10b-hexahydro-phenanthridine
(4aR,10bR)-6-(4,6-Diethoxy-pyridin-3-yl)-9-ethoxy-8-methoxy-1,2,3,4,4a,10b-hexahydro-phenanthridine
(4aR,10bR)-6-(5,6-Dimethoxy-pyridin-3-yl)-9-ethoxy-8-methoxy-1,2,3,4,4a,10b-hexahydro-phenanthridine
(4aR,10bR)-6-(5-Ethoxy-6-methoxy-pyridin-3-yl)-9-ethoxy-8-methoxy-1,2,3,4,4a,10b-hexahydro-phenanthridine
(4aR,10bR)-6-(5-Ethoxy-6-methoxy-pyridin-3-yl)-9-ethoxy-8-methoxy-1,2,3,4,4a,10b-hexahydro-phenanthridine
5-((4aR,10bR)-9-Ethoxy-8-methoxy-1,2,3,4,4a,10b-hexahydro-phenanthridin-6-yl)-1-methyl-1H-pyridin-2-one

### Starting compounds:

**A1. N-[(1R,2R)-2-(3,4-Dimethoxy-phenyl)-cyclohexyl]-2,6-dimethoxy-nicotinamide**
2.00 g (1R,2R)-2-(3,4-dimethoxyphenyl)-cyclohexylamine (compound B1), 2346 mg 1-ethyl-3-(3-dimethylaminopropyl)carbodiimide hydrochloride and 1868 mg of 2,6-dimethoxynicotinic acid are dissolved in 4 ml of dichloromethane. 4 mg of *p*-dimethylaminopyridine are added and the reaction mixture is stirred for 16 h. After work-up (washing consecutively with water and sat. NaHCO₃) the residue is purified by means of chromatography to yield 4.07 g of the title compound which is used for the following step without further purification.

| | | |
|---|---|---|
| Calc. C₂₂ H₂₈ N₂ O₅ | (400.48) | found MH+: 401.1 |

Starting from the appropriate amine mentioned below or obtainable analogously or similarly to the below-described compounds and the appropriate pyridine-carboxylic acid the following and further compounds of this invention can be obtained according to compound A1.
**A2. N-[(1R,2R)-2-(3-Ethoxy-4-methoxy-phenyl)-cyclohexy]l-2,6-dimethoxy-nicotinamide**

| | |
|---|---|
| Calc. C₂₃ H₃₀ N₂ O₅ | (414.51) |

**A3. 3,5-Dichloro-N-[(1R,2R)-2-(3,4-dimethoxy-phenyl)-cyclohexyl]-isonicotinamide**
**B1. (1R,2R)-2-(3,4-Dimethoxyphenyl)-cyclohexylamine**
12.0 g of a racemic mixture of (1R,2R)-2-(3,4-dimethoxyphenyl)-cyclohexylamine and (1S,2S)-2-(3,4-dimethoxyphenyl)-cyclohexylamine and 6.2 g of (-)-mandelic acid are dissolved in 420 ml of dioxane and 60 ml of tetrahydrofuran and the solution is stirred overnight at RT. The solid is filtered off with suction, dried, treated with 100 ml of saturated sodium hydrogencarbonate solution and extracted with ethyl acetate. The organic phase is dried using sodium sulfate and concentrated under reduced pressure. 4.8 g of the title compound are obtained of m.p.: 80-81.5°C.
Specific rotation: [α]²⁰*_{D}* = -58.5°C (c = 1, ethanol).
The following compound and further relevant starting compounds can be obtained analogously as described for compound B1.
**B2. (1R,2R)-2-(3-Ethoxy-4-methoxy-phenyl)-cyclohexylamine**
**C1. Racemic mixture of (1R,2R)-2-(3,4-dimethoxyphenyl)-cyclohexylamine and (1S,2S)-2-(3,4-dimethoxyphenyl)-cyclohexylamine**
125 g of a racemic mixture of 1,2-dimethoxy-4-((1R,2R)-2-nitrocyclohexyl)benzene and 1,2-dimethoxy-4-((1S,2S)-2-nitrocyclohexyl)benzene and 120 g of zinc powder or granules are suspended in 1300 ml of ethanol. 220 ml of acetic acid are added dropwise at boiling heat. The precipitate is filtered off with suction and washed with ethanol, and the filtrate is concentrated under reduced pressure. The residue is taken up in hydrochloric acid and extracted with toluene. The aqueous phase is rendered alkaline using 50% strength sodium hydroxide solution, the precipitate is filtered off with suction and the filtrate is extracted with toluene. The organic phase is dried using sodium sulfate and concentrated. 98 g of the title compound are obtained as a crystallizing oil.
**Alternatively:**
8.5 g of a racemic mixture of 1,2-dimethoxy-4-((1R,2R)-2-nitrocyclohexyl)benzene and 1,2-dimethoxy-4-((1S,2S)-2-nitrocyclohexyl)benzene are dissolved in 400 ml of methanol and treated at RT with 7 ml of hydrazine hydrate and 2.5 g of Raney nickel in portions in the course of 8 h. After stirring overnight at RT, the reaction mixture is filtered, the filtrate is concentrated and the residue is chromatographed on silica gel using a mixture of toluene/ethyl acetate/triethylamine = 4/2/0.5. The title compound is obtained as an oil.
**D1. Racemic mixture of 1,2-dimethoxy-4-((1R,2R)-2-nitrocyclohexyl)benzene and 1,2-dimethoxy-4-((1S,2S)-2-nitrocyclohexyl)benzene**
8.4 g of a racemic mixture of 1,2-dimethoxy-4-((1R,2R)-2-nitrocyclohex-4-enyl)benzene and 1,2-dimethoxy-4-((1S,2S)-2-nitrocyclohex-4-enyl)benzene are dissolved in 450 ml of methanol, treated with 2 ml of conc. hydrochloric acid and hydrogenated after addition of 500 mg of 10% strength Pd/C. The reaction mixture is filtered and the filtrate is concentrated. M.p.: 84-86.5°C.
**E1. Racemic mixture of 1,2-dimethoxy-4-((1R,2R)-2-nitrocyclohex-4-enyl)benzene and 1,2-dimethoxy-4-((1S,2S)-2-nitrocyclohex-4-enyl)benzene**
10.0 g of a racemic mixture of 1,2-dimethoxy-4-((1R,2S)-2-nitrocyclohex-4-enyl)benzene and 1,2-dimethoxy-4-((1S,2R)-2-nitrocyclohex-4-enyl)benzene and 20.0 g of potassium hydroxide are dissolved in 150 ml of ethanol and 35 ml of dimethylformamide. A solution of 17.5 ml of conc. sulfuric acid in 60 ml of ethanol is then added dropwise such that the internal temperature does not exceed 4°C. After stirring for 1 h, the mixture is added to 1 l of ice water, the precipitate is filtered off with suction, washed with water and dried, and the crude product is recrystallized from ethanol. 8.6 g of the title compound of m.p. 82.5-84°C are obtained.
**F1. Racemic mixture of 1,2-dimethoxy-4-((1R,2S)-2-nitrocyclohex-4-enyl)benzene and 1,2-dimethoxy-4-((1S,2R)-2-nitrocyclohex-4-enyl)benzene**
50.0 g of 3,4-dimethoxy-ω-nitrostyrene (compound G1) and 1.0 g (9.1 mmol) of hydroquinone are suspended in 200 ml of dry toluene and treated at -70°C with 55.0 g (1.02 mol) of liquid 1,3-butadiene. The mixture is stirred at 160°C for 6 days in an autoclave and then cooled. Some of the solvent is removed on a rotary evaporator, and the resulting precipitate is filtered off with suction and recrystallized in ethanol. M.p.: 113.5-115.5°C.
**G1. 3,4-Dimethoxy-**ω**-nitrostyrene**
207.0 g of 3,4-dimethoxybenzaldehyde, 100.0 g of ammonium acetate and 125 ml of nitromethane are heated to boiling for 3-4 h in 1.0 I of glacial acetic acid. After cooling in an ice bath, the precipitate is filtered off with suction, rinsed with glacial acetic acid and petroleum ether and dried. M.p.: 140-141 °C. Yield: 179.0 g.
Starting from starting compounds, which are art-known or which can be obtained analogously to art-known compounds or according to art-known procedures (such as e.g. as described in WO 95/01338 or analogously or similarly thereto) the following compounds are obtained according to the procedure as in Example G1:
**G2. 3-Ethoxy-4-methoxy-**ω**-nitrostyrene**
**G3. 4-(1,1-Difluoro-methoxy)-3-methoxy-**ω**-nitrostyrene**
**G4. 3-(1,1-Difluoro-methoxy)-4-methoxy-**ω**-nitrostyrene**

### Commercial utility

The compounds according to the invention have useful pharmacological properties which make them industrially utilizable. As selective cyclic nucleotide phosphodiesterase (PDE) inhibitors (specifically of type 4), they are suitable on the one hand as bronchial therapeutics (for the treatment of airway obstructions on account of their dilating action but also on account of their respiratory rate- or respiratory drive-increasing action) and for the removal of erectile dysfunction on account of their vascular dilating action, but on the other hand especially for the treatment of disorders, in particular of an inflammatory nature, e.g. of the airways (asthma prophylaxis), of the skin, of the intestine, of the eyes, of the CNS and of the joints, which are mediated by mediators such as histamine, PAF (platelet-activating factor), arachidonic acid derivatives such as leukotrienes and prostaglandins, cytokines, interleukins, chemokines, alpha-, beta- and gamma-interferon, tumor necrosis factor (TNF) or oxygen free radicals and proteases. In this context, the compounds according to the invention are distinguished by a low toxicity, a good enteral absorption (high bioavailability), a large therapeutic breadth and the absence of significant side effects.

On account of their PDE-inhibiting properties, the compounds according to the invention can be employed in human and veterinary medicine as therapeutics, where they can be used, for example, for the treatment and prophylaxis of the following illnesses: acute and chronic (in particular inflammatory and allergen-induced) airway disorders of varying origin (bronchitis, allergic bronchitis, bronchial asthma, emphysema, COPD); dermatoses (especially of proliferative, inflammatory and allergic type) such as psoriasis (vulgaris), toxic and allergic contact eczema, atopic eczema, seborrhoeic eczema, Lichen simplex, sunburn, pruritus in the anogenital area, alopecia areata, hypertrophic scars, discoid lupus erythematosus, follicular and widespread pyodermias, endogenous and exogenous acne, acne rosacea and other proliferative, inflammatory and allergic skin disorders; disorders which are based on an excessive release of TNF and leukotrienes, for example disorders of the arthritis type (rheumatoid arthritis, rheumatoid spondylitis, osteoarthritis and other arthritic conditions), disorders of the immune system (AIDS, multiple sclerosis), graft versus host reaction, allograft rejections, types of shock (septic shock, endotoxin shock, gram-negative sepsis, toxic shock syndrome and ARDS (adult respiratory distress syndrome)) and also generalized inflammations in the gastrointestinal region (Crohn's disease and ulcerative colitis); disorders which are based on allergic and/or chronic, immunological false reactions in the region of the upper airways (pharynx, nose) and the adjacent regions (paranasal sinuses, eyes), such as allergic rhinitis/sinusitis, chronic rhinitis/sinusitis, allergic conjunctivitis and also nasal polyps; but also disorders of the heart which can be treated by PDE inhibitors, such as cardiac insufficiency, or disorders which can be treated on account of the tissue-relaxant action of the PDE inhibitors, such as, for example, erectile dysfunction or colics of the kidneys and of the ureters in connection with kidney stones. In addition, the compounds of the invention are useful in the treatment of diabetes insipidus and conditions associated with cerebral metabolic inhibition, such as cerebral senility, senile dementia (Alzheimer's disease), memory impairment associated with Parkinson's disease or multiinfarct dementia; and also illnesses of the central nervous system, such as depressions or arteriosclerotic dementia; as well as for enhancing cognition. Yet in addition, the compounds of the invention are useful in the treatment of diabetes mellitus, leukaemia and osteoporosis.

The invention further relates to the compounds according to the invention for use in the treatment and/or prophylaxis of illnesses, especially the illnesses mentioned.

The invention also relates to the use of the compounds according to the invention for the production of pharmaceutical compositions which are employed for the treatment and/or prophylaxis of the illnesses mentioned.

The invention also relates to the use of the compounds according to the invention for the production of pharmaceutical compositions for treating disorders which are mediated by phosphodiesterases, in particular PDE4-mediated disorders, such as, for example, those mentioned in the specification of this invention or those which are apparent or known to the skilled person.

The invention also relates to the use of the compounds according to the invention for the manufacture of pharmaceutical compositions having PDE4 inhibitory activity.

The invention furthermore relates to pharmaceutical compositions for the treatment and/or prophylaxis of the illnesses mentioned comprising one or more of the compounds according to the invention.

The invention yet furthermore relates to compositions comprising one or more compounds according to this invention and pharmaceutically acceptable auxiliaries and/or excipients.

The invention yet furthermore relates to compositions comprising one or more compounds according to this invention and a pharmaceutically acceptable carrier. Said compositions can be used in therapy, such as e.g. for treating, preventing or ameliorating one or more of the abovementioned diseases.

The invention still yet furthermore relates to pharmaceutical compositions according to this invention having PDE, particularly PDE4, inhibitory activity.

The pharmaceutical compositions are prepared by processes which are known per se and familiar to the person skilled in the art. As pharmaceutical compositions, the compounds according to the invention (= active compounds) are either employed as such, or preferably in combination with suitable pharmaceutical auxiliaries and/or excipients, e.g. in the form of tablets, coated tablets, capsules, caplets, suppositories, patches (e.g. as TTS), emulsions, suspensions, gels or solutions, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries, excipients, carriers, vehicles, diluents or adjuvants which are suitable for the desired pharmaceutical formulations on account of his/her expert knowledge. In addition to solvents, gel formers, ointment bases and other active compound excipients, for example antioxidants, dispersants, emulsifiers, preservatives, solubilizers, colorants, complexing agents or permeation promoters, can be used.

The administration of the pharmaceutical compositions according to the invention may be performed in any of the generally accepted modes of administration available in the art. Illustrative examples of suitable modes of administration include intravenous, oral, nasal, parenteral, topical, transdermal and rectal delivery. Oral delivery is preferred.

For the treatment of disorders of the respiratory tract, the compounds according to the invention are preferably also administered by inhalation in the form of an aerosol; the aerosol particles of solid, liquid or mixed composition preferably having a diameter of 0.5 to 10 µm, advantageously of 2 to 6 µm.

Aerosol generation can be carried out, for example, by pressure-driven jet atomizers or ultrasonic atomizers, but advantageously by propellant-driven metered aerosols or propellant-free administration of micronized active compounds from inhalation capsules.

Depending on the inhaler system used, in addition to the active compounds the administration forms additionally contain the required excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of apparatuses are available with which aerosols of optimum particle size can be generated and administered, using an inhalation technique which is as right as possible for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhaler described in European Patent Application EP 0 505 321), using which an optimal administration of active compound can be achieved.

For the treatment of dermatoses, the compounds according to the invention are in particular administered in the form of those pharmaceutical compositions which are suitable for topical application. For the production of the pharmaceutical compositions, the compounds according to the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, pastes, gels or solutions.

The pharmaceutical compositions according to the invention are prepared by processes known per se. The dosage of the active compounds is carried out in the order of magnitude customary for PDE inhibitors. Topical application forms (such as ointments) for the treatment of dermatoses thus contain the active compounds in a concentration of, for example, 0.1-99%. The dose for administration by inhalation is customarly between 0.01 and 3 mg per day. The customary dose in the case of systemic therapy (p.o. or i.v.) is between 0.003 and 3 mg/kg per day. In another embodiment, the dose for administration by inhalation is between 0.1 and 3 mg per day, and the dose in the case of systemic therapy (p.o. or i.v.) is between 0.03 and 3 mg/kg per day.

### Biological investigations

The second messenger cyclic AMP (cAMP) is well-known for inhibiting inflammatory and immunocompetent cells. The PDE4 isoenzyme is broadly expressed in cells involved in the initiation and propagation of inflammatory diseases (H Tenor and C Schudt, in "Phosphodiesterase Inhibitors", 21-40, "The Handbook of Immunopharmacology", Academic Press, 1996), and its inhibition leads to an increase of the intracellular cAMP concentration and thus to the inhibition of cellular activation (JE Souness et al., Immunopharmacology 47: 127-162, 2000).

The antiinflammatory potential of PDE4 inhibitors in vivo in various animal models has been described (MM Teixeira, TiPS 18: 164-170, 1997). For the investigation of PDE4 inhibition on the cellular level (in vitro), a large variety of proinflammatory responses can be measured. Examples are the superoxide production of neutrophilic (C Schudt et al., Arch Pharmacol 344: 682-690, 1991) or eosinophilic (A Hatzelmann et al., Brit J Pharmacol 114: 821-831, 1995) granulocytes, which can be measured as luminol-enhanced chemiluminescence, or the synthesis of tumor necrosis factor-α in monocytes, macrophages or dendritic cells (Gantner et al., Brit J Pharmacol 121: 221-231, 1997, and Pulmonary Pharmacol Therap 12: 377-386, 1999). In addition, the immunomodulatory potential of PDE4 inhibitors is evident from the inhibition of T-cell responses like cytokine synthesis or proliferation (DM Essayan, Biochem Pharmacol 57: 965-973, 1999). Substances which inhibit the secretion of the afore-mentioned proinflammatory mediators are those which inhibit PDE4. PDE4 inhibition by the compounds according to the invention is thus a central indicator for the suppression of inflammatory processes.

### Methods for measuring inhibition of PDE4 activity

The PDE4B2 (GB no. M97515) was a gift of Prof. M. Conti (Stanford University, USA). It was amplified from the original plasmid (pCMV5) via PCR with primers Rb9 (5'- GCCAGCGTGCAAATAATGAAGG -3') and Rb10 (5'- AGAGGGGGATTATGTATCCAC -3') and cloned into the pCR-Bac vector (Invitrogen, Groningen, NL).

The recombinant baculovirus was prepared by means of homologous recombination in SF9 insect cells. The expression plasmid was cotransfected with Bac-N-Blue (Invitrogen, Groningen, NL) or Baculo-Gold DNA (Pharmingen, Hamburg) using a standard protocol (Pharmingen, Hamburg). Wt virus-free recombinant virus supernatant was selected using plaque assay methods. After that, high-titre virus supernatant was prepared by amplifying 3 times. PDE was expressed in SF21 cells by infecting 2×10⁶ cells/ml with an MOI (multiplicity of infection) between 1 and 10 in serum-free SF900 medium (Life Technologies, Paisley, UK). The cells were cultured at 28°C for 48 - 72 hours, after which they were pelleted for 5-10 min at 1000 g and 4°C.

The SF21 insect cells were resuspended, at a concentration of approx. 10⁷ cells/ml, in ice-cold (4°C) homogenization buffer (20 mM Tris, pH 8.2, containing the following additions: 140 mM NaCl, 3.8 mM KCI, 1 mM EGTA, 1 mM MgCl₂, 10 mM β-mercaptoethanol, 2 mM benzamidine, 0.4 mM Pefablock, 10 µM leupeptin, 10 µM pepstatin A, 5 µM trypsin inhibitor) and disrupted by ultrasonication. The homogenate was then centrifuged for 10 min at 1000xg and the supernatant was stored at -80°C until subsequent use (see below). The protein content was determined by the Bradford method (BioRad, Munich) using BSA as the standard.

PDE4B2 activity is inhibited by the said compounds in a modified SPA (scintillation proximity assay) test, supplied by Amersham Biosciences (see procedural instructions "phosphodiesterase [3H]cAMP SPA enzyme assay, code TRKQ 7090"), carried out in 96-well microtitre plates (MTP's). The test volume is 100 µl and contains 20 mM Tris buffer (pH 7.4), 0.1 mg of BSA (bovine serum albumin)/ml, 5 mM Mg²⁺, 0.5 µM cAMP (including about 50,000 cpm of [3H]cAMP), 1 µl of the respective substance dilution in DMSO and sufficient recombinant PDE (1000xg supernatant, see above) to ensure that 10-20% of the cAMP is converted under the said experimental conditions. The final concentration of DMSO in the assay (1 % v/v) does not substantially affect the activity of the PDE investigated. After a preincubation of 5 min at 37°C, the reaction is started by adding the substrate (cAMP) and the assay is incubated for a further 15 min; after that, it is stopped by adding SPA beads (50 µl). In accordance with the manufacturer's instructions, the SPA beads had previously been resuspended in water, but were then diluted 1:3 (v/v) in water; the diluted solution also contains 3 mM IBMX to ensure a complete PDE activity stop. After the beads have been sedimented (> 30 min), the MTP's are analyzed in commercially available luminescence detection devices. The corresponding IC₅₀ values of the compounds for the inhibition of PDE activity are determined from the concentration-effect curves by means of non-linear regression.

A representative inhibitory value determined for the compounds according to the invention follow from the following table A, in which the number of the compound correspond to the numbers of the Example.

**Table A**

| Inhibition of the PDE4 activity | |
|---|---|
| Compound | -log IC₅₀ (mol/l) |
| 2 | 8.49 |

## Claims

1. Compounds of the formula I, 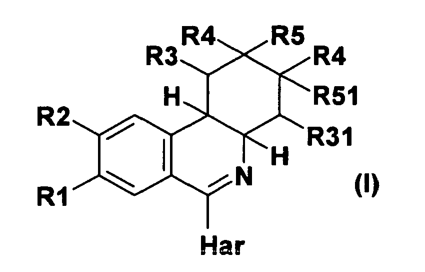 in which
R1 is methoxy or ethoxy,
R2 is methoxy, ethoxy or difluoromethoxy,
R3, R31, R4, R5 and R51 are hydrogen,
Har is selected from 2,6-dimethoxypyridin-4-yl, 2,6-dimethoxypyridin-3-yl, 4,6-dimethoxy-pyridin-3-yl, 4,6-diethoxy-pyridin-3-yl, 5,6-dimethoxy-pyridin-3-yl, 5-ethoxy-6-methoxy-pyridin-3-yl and 1-methyl-1H-pyridin-2-one-5-yl,
and the salts, the N-oxides and the salts of the N-oxides of these compounds.

2. Compounds of the formula I as claimed in claim 1 selected from
(4aR,10bR)-6-(2,6-Dimethoxy-pyridin-3-yl)-8,9-dimethoxy-1,2,3,4,4a,10b-hexahydro-phenanthridine,
(4aR,10bR)-6-(2,6-Dimethoxy-pyridin-3-yl)-9-ethoxy-8-methoxy-1,2,3,4,4a,10b-hexahydro-phenanthridine,
and the salts, the N-oxides and the salts of the N-oxides of these compounds.

3. Compounds of the formula I according to claim 1, which have with respect to the positions 4a and 10b the configuration shown in formula I*: 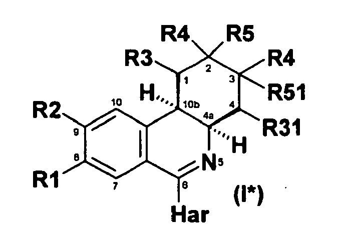 and the salts, the N-oxides and the salts of the N-oxides of these compounds.

4. Compounds, salts, N-oxides or salts of the N-oxides as claimed in any of the claims 1 to 3 for use in the treatment or prevention of diseases.

5. A pharmaceutical composition comprising one or more compounds, salts, N-oxides or salts of the N-oxides as claimed in any of the claims 1 to 3 together with a pharmaceutical auxiliary and/or excipient.

6. The use of compounds, salts, N-oxides or salts of the N-oxides as claimed in any of the claims 1 to 3 for the production of pharmaceutical compositions for treating respiratory disorders.

## Patentansprüche

1. Verbindungen der Formel I 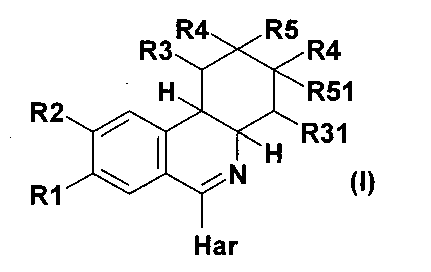 in welcher
R1 für Methoxy oder Ethoxy steht,
R2 für Methoxy, Ethoxy oder Difluormethoxy steht,
R3, R31, R4, R5 und R51 für Wasserstoff stehen,
Har ausgewählt ist aus 2,6-Dimethoxypyridin-4-yl, 2,6-Dimethoxypyridin-3-yl, 4,6-Dimethoxypyridin-3-yl, 4,6-Diethoxypyridin-3-yl, 5,6-Dimethoxypyridin-3-yl, 5-Ethoxy-6-methoxypyridin-3-yl und 1-Methyl-1H-pyridin-2-on-5-yl,
und die Salze, die N-Oxide und die Salze der N-Oxide dieser Verbindungen.

2. Verbindungen der Formel I nach Anspruch 1, ausgewählt aus
(4aR,10bR)-6-(2,6-Dimethoxypyridin-3-yl)-8,9-dimethoxy-1,2,3,4,4a,10b-hexahydrophenanthridin,
(4aR,10bR)-6-(2,6-Dimethoxypyridin-3-yl)-9-ethoxy-8-methoxy-1,2,3,4,4a,10b-hexahydrophenanthridin
und die Salze, die N-Oxide und die Salze der N-Oxide dieser Verbindungen.

3. Verbindungen der Formel I nach Anspruch 1, die, bezüglich der Positionen 4a und 10b, die in Formel I* gezeigte Konfiguration aufweisen: 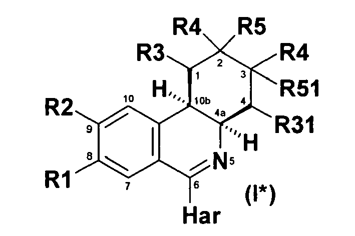 und die Salze, die N-Oxide und die Salze der N-Oxide dieser Verbindungen.

4. Verbindungen, Salze, N-Oxide oder Salze der N-Oxide nach einem der Ansprüche 1 bis 3 zur Verwendung bei der Behandlung oder Prävention von Krankheiten.

5. Pharmazeutische Zusammensetzung, enthaltend ein(e) oder mehrere Verbindungen, Salze, N-Oxide oder Salze der N-Oxide nach einem der Ansprüche 1 bis 3 zusammen mit einem pharmazeutischen Hilfsstoff und/oder Exzipienten.

6. Verwendung der Verbindungen, Salze, N-Oxide oder Salze der N-Oxide nach einem der Ansprüche 1 bis 3 zur Herstellung von pharmazeutischen Zusammensetzungen zur Behandlung von Atemwegserkrankungen.

## Revendications

1. Composés de formule I, 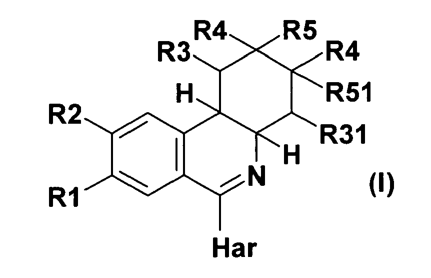 dans laquelle
R1 est méthoxy ou éthoxy,
R2 est méthoxy, éthoxy ou difluorométhoxy,
R3, R31, R4, R5 et R51 sont hydrogène,
Har est choisi parmi 2,6-diméthoxypyridin-4-yle, 2,6-diméthoxypyridin-3-yle, 4,6-diméthoxypyridin-3-yle, 4,6-diéthoxypyridin-3-yle, 5,6-diméthoxypyridin-3-yle, 5-éthoxy-6-méthoxypyridin-3-yle et 1-méthyl-1H-pyridin-2-on-5-yle,
et les sels, les N-oxydes et les sels des N-oxydes de ces composés.

2. Composés de formule I selon la revendication 1, choisis parmi
la (4aR,10bR)-6-(2,6-diméthoxypyridin-3-yl)-8,9-diméthoxy-1,2,3,4,4a,10b-hexahydrophénanthridine,
la (4aR,10bR)-6-(2,6-diméthoxypyridin-3-yl)-9-éthoxy-8-méthoxy-1,2,3,4,4a,10b-hexahydrophénanthridine,
et les sels, les N-oxydes et les sels des N-oxydes de ces composés.

3. Composés de formule I selon la revendication 1, **caractérisés en ce qu'**ils ont, par rapport aux positions 4a et 10b, la configuration présentée dans la formule I* : 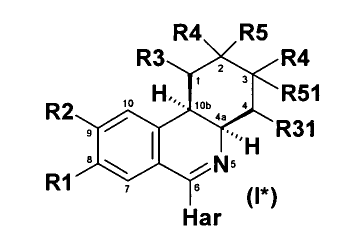 et les sels, les N-oxydes et les sels des N-oxydes de ces composés.

4. Composés, sels, N-oxydes ou sels des N-oxydes selon l'une quelconque des revendications 1 à 3, destinés à être utilisés dans le traitement ou la prévention de maladies.

5. Composition pharmaceutique comprenant un ou plusieurs composés, sels, N-oxydes ou sels des N-oxydes selon l'une quelconque des revendications 1 à 3, conjointement avec un auxiliaire et/ou un excipient pharmaceutique.

6. Utilisation de composés, sels, N-oxydes ou sels des N-oxydes selon l'une quelconque des revendications 1 à 3, pour la production de compositions pharmaceutiques destinées au traitement de troubles respiratoires.
